(19) Europäisches Patentamt
European Patent Office
Office européen des brevets

(11) Veröffentlichungsnummer : **0 051 597**
**B1**

(12) # EUROPÄISCHE PATENTSCHRIFT

(45) Veröffentlichungstag der Patentschrift : **04.07.84**

(51) Int. Cl.³ : **C 07 D307/93**, A 61 K 31/34

(21) Anmeldenummer : **81900445.8**

(22) Anmeldetag : **05.02.81**

(86) Internationale Anmeldenummer :
**PCT/DE 81/00031**

(87) Internationale Veröffentlichungsnummer :
**WO/8102296 (20.08.81 Gazette 81/20)**

(54) **NEUE PROSTACYCLINDERIVATE UND IHRE HERSTELLUNG.**

(30) Priorität : **07.02.80 DE 3004795**

(43) Veröffentlichungstag der Anmeldung :
**19.05.82 Patentblatt 82/20**

(45) Bekanntmachung des Hinweises auf die Patenterteilung : **04.07.84 Patentblatt 84/27**

(84) Benannte Vertragsstaaten :
**AT CH DE FR GB LI LU NL SE**

(56) Entgegenhaltungen :
**EP-A- 0 002 234**
**GB-A- 1 493 557**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **VORBRÜGGEN, Helmut**
**Wilkestrasse 7**
**D-1000 Berlin 27 (DE)**
Erfinder : **RADÜCHEL, Bernd**
**Gollanczstrasse 132**
**D-1000 Berlin 28 (DE)**
Erfinder : **SKUBALLA, Werner**
**Olwenstrasse 25**
**D-1000 Berlin 28 (DE)**
Erfinder : **MANNESMANN, Gerda**
**Hagenplatz 3e**
**D-1000 Berlin 33 (DE)**
Erfinder : **CASALS-STENZEL, Jorge**
**Wichernstrasse 20**
**D-1000 Berlin 33 (DE)**
Erfinder : **SCHILLINGER, Ekkehard**
**Im Amseltal 50**
**D-1000 Berlin 28 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 051 597 B1

Jouve, 18, rue St-Denis, 75001 Paris, France

**Beschreibung**

Die Erfindung betrifft neue Prostacyclin-Derivate, Verfahren zu ihrer Herstellung sowie ihre Eignung als Arzneimittel.

Prostacyclin ($PGI_2$), einer der Hauptfaktoren bei der Blutplättchenaggregation, wirkt dilatierend auf verschiedene Blutgefäße (Science 196, 1072) und ist daher als Mittel zur Blutdrucksenkung anzusehen. $PGI_2$ besitzt jedoch nicht die für ein Arzneimittel notwendige Stabilität. So beträgt die Halbwertzeit von $PGI_2$ bei physiologischen pH-Werten und bei Raumtemperatur nur wenige Minuten. Durch die Einführung einer Nitrilgruppe an die Enolätherdoppelbindung wird das Prostacyclin stabilisiert, wobei das pharmakologische Wirkungsspektrum erhalten bleibt und die Wirkungsdauer deutlich verlängert wird (DE-OS 2 753 244). Die zusätzliche Einführung von Fluor in 16-Stellung bewirkt eine weitere Steigerung der Wirkungsdauer und Selektivität.

Die erfindungsgemäßen Verbindungen wirken blutdrucksenkend und bronchodilatorisch. Außerdem sind diese Verbindungen zur Inhibierung der Thrombozytenaggregation und zur Hemmung der Magensäuresekretion geeignet. Sie zeigen überraschende Aktivitäten im Vergleich zu in EP-A-2234 und GB-A-1493 557 beschriebenen fluorierten Prostaglandinverbindungen.

Die Erfindung betrifft Prostanderivate der allgemeinen Formel I

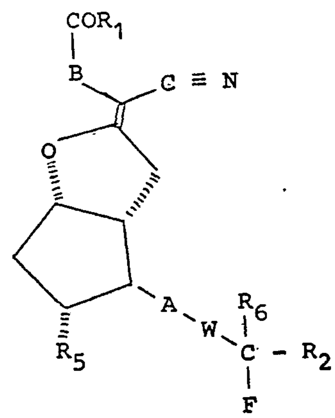

(I)

worin

$R_1$ den Rest $OR_3$, wobei $R_3$ Wasserstoff oder Alkyl mit 1-10 C-Atomen bedeuten kann, oder $R_1$ den Rest $NHR_4$ mit $R_4$ in der Bedeutung eines Alkanoyl- oder Alkansulfonylrestes mit jeweils 1-4 C-Atomen darstellt,

B eine geradkettige oder verzweigtkettige Alkylengruppe mit 1-5 C-Atomen,

A eine $-CH_2-CH_2-$, cis-CH=CH- oder trans-CH=CH-Gruppe,

W eine Hydroxymethylengruppe oder eine

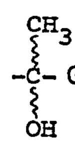 Gruppe

in denen die OH-Gruppe jeweils durch Alkanoyl mit 1-4-C-Atomen substituiert sein kann, wobei die freie oder substituierte OH-Gruppe α- oder β-ständig sein kann,

$R_2$ eine gesättigte gerad- oder verzweigtkettige Alkylgruppe mit 1-6 C-Atomen, die durch Phenyl substituiert sein kann oder eine Phenylgruppe,

$R_5$ eine Hydroxygruppe, die durch Alkanoyl mit 1-4 C-Atomen substituiert sein kann,

$R_6$ Wasserstoff, Fluor oder Alkyl mit 1-4 C-Atomen und falls $R_3$ Wasserstoff darstellt, deren Salze mit physiologisch verträglichen Basen bedeuten.

Als Alkylgruppe $R_3$ sind geradkettige oder verzweigtkettige Alkylgruppen mit 1-10 C-Atomen zu betrachten, wie beispielsweise Methyl, Äthyl, Propyl, Butyl, Isobutyl, tert.-Butyl, Pentyl, Neopentyl, Heptyl, Hexyl, Decyl. Als bevorzugte Alkylgruppen $R_3$ sind solche mit 1-4 C-Atomen, wie z. B. Methyl, Äthyl, Propyl, Isobutyl, Butyl zu nennen.

Als Alkanoylrest $R_4$ kommen physiologisch verträgliche Säurereste in Frage, die sich von folgenden Carbonsäuren ableiten : Ameisensäure, Essigsäure, Propionsäure, Buttersäure oder Isobuttersäure. Die Alkansulfonylreste $R_4$ leiten sich beispeilsweise von Methansulfonsäure, Äthansulfonsäure, Isopropansulfonsäure, Butansulfonsäure ab.

Als Alkanoylreste in $R_5$ und W seien namentlich Acetyl, Propionyl und Butyryl genannt. Als Alkylgruppe $R_2$ kommen geradkettige und verzweigtkettige, gesättigte Alkylreste mit 1-6 C-Atomen in Frage, die durch Phenyl substituiert sein können. Beispielsweise genannt seien Methyl-, Äthyl-, Propyl-, Butyl-, Isobutyl-, tert.-Butyl-, Pentyl-, Hexyl- und Benzyl-.

Als Alkylgruppe $R_6$ kommen gerad- und verzweigtkettige, gesättigte Alkylreste mit 1-4 C-Atomen in Frage. Namentlich seien genannt Methyl, Äthyl, Propyl, Isopropyl, Butyl, Isobutyl, tert.-Butyl.

Als Alkylengruppe B kommen geradkettige gesättigte Alkylenreste mit 1-5 C-Atomen in Frage. Namentlich seien genannt Methylen, Äthylen, Trimethylen, Tetramethylen, Pentamethylen.

Zur Salzbildung sind anorganische und organische Basen geeignet, wie sie dem Fachmann zur Bildung physiologisch verträglicher Salze bekannt sind. Beispielsweise seien genannt Alkylihydroxide, wie Natrium und Kaliumhydroxid, Erdalkalihydroxide, wie Calciumhydroxid, Ammoniak, amine, wie Äthanolamin, Diäthanolamin, Triäthanolamin, N-Methylglucamin, Morpholin, Tris-(hydroxymethyl)-methylamin usw.

Die Erfindung betrifft ferner ein Verfahren zur Herstellung der erfindungsgemäßen Prostacyclinderivate der allgemeinen Formel I, dadurch gekennzeichnet, daß man in an sich bekannter Weise eine Verbindung der allgemeinen Formel II

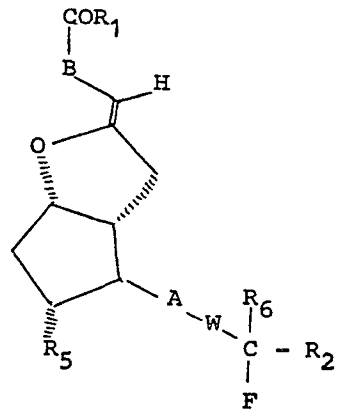

(II)

worin $R_1$, $R_2$, $R_5$, $R_6$, A, B und W die obenangegebenen Bedeutungen haben, gegebenenfalls nach Schutz anwesender freier Hydroxygruppen, haben, gegebenenfalls nach Schutz anwesender freier Hydroxygruppen, mit einem Sulfonylisocyanat der allgemeinen Formel III,

$$R_7SO_2NCO \qquad (III)$$

worin $R_7$ einen gegebenenfalls durch Alkyl mit 1-4 C-Atomen substituierten Phenylring, halogeniertes Alkyl oder Halogen bedeutet, und anschließend mit einem tertiären Amin oder tertiären Amid umsetzt.

Gegebenenfalls kann man anschließend in den erhaltenen Verfahrensprodukten in beliebiger Reihenfolge geschützte Hydroxygruppen freisetzen und/oder freie Hydroxygruppen verestern, veräthern oder oxydieren und die erhaltenen Carbonylgruppen mit Methylmagnesiumhalogeniden umsetzen, eine veresterte Carbonylgruppe verseifen oder eine Carbonylgruppe verestern, eine Carboxylgruppe mit Verbindungen der allgemeinen Formel IV

$$O=C=N—R_4 \qquad (IV)$$

worin $R_4$, die obenangegebene Bedeutung hat, umsetzen oder eine Carboxylgruppe mit einer physiologisch verträglichen Base in ein Salz überführen.

Die Umsetzung der Enoläther II zu den Verbindungen der allgemeinen Formel I erfolgt nacheinander im gleichen Reaktionsgefäß mit Sulfonylisocyanten der Formel III und einem tertiären Amin in einem inerten Lösungsmittel, vorzugsweise Tetrahydrofuran, Dioxan, Diäthyläther, Toluol bzw. tert. Amid vorzugsweise ohne Lösungsmittel. Unter den Sulfonylisocyanaten der allgemeinen Formel III seien genannt Benzolsulfonylisocyanat, p-Toluolsulfonylisocyanat sowie die Halogensulfonylisocyanate mit den Halogenen Fluor, Chlor und Brom. Besonders bevorzugt für die Reaktion mit Verbindungen der Formel II ist das Chlorsulfonylisocyanat.

Die Reaktion mit Sulfonylisocyanaten der Formel III erfolgt bei Temperaturen zwischen 30 °C und − 100 °C, vorzugsweise zwischen − 70 °C und 0 °C. Die Umsetzung mit einem tertiären Amin oder tertiären Amid erfolgt bei Temperaturen zwischen − 100 °C und 30 °C, vorzugsweise zwischen − 70 °C und + 30 °C. Als tertiäre Amine kommen beispielsweise in Frage : Triäthylamin, Trimethylamin, Diäthylisopropylamin, Dimethylisopropylamin, DBN, DBU usw.

Als tertiäres Amid kommt vorzugsweise Dimethylformamid in Frage.

Die Verseifung der Prostacyclinester wird nach den dem Fachmann bekannten Methoden durchgeführt, wie beispielsweise mit basischen Katalysatoren.

Die Einführung der Estergruppe —$OR_3$ für $R_1$, bei welcher $R_3$ eine Alkylgruppe mit 1-10 C-Atom darstellt, erfolgt nach den dem Fachman bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit der Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach den bekannten Methoden hergestellt werden [Org. Reactions Bd. 8, Seiten 389-394 (1954)].

Die Prostacyclin-Derivate der allgemeinen Formel I mit $R_1$ in der Bedeutung einer Hydroxygruppe können mit geeigneten Mengen der entsprechenden anorganischen Basen unter Neutralisierung in Salze überführt werden. Beispielsweise erhält man beim Lösen der entsprechenden Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Zur Herstellung eines Aminsalzes, die in üblicher Weise erfolgt, wird die Säure zum Beispiel in einem geeigneten Lösungsmittel, beispielsweise Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u. a., umsetzt.

Die Freisetzung einer funktionell abgewandelten OH-Gruppe zu den Verbindungen der allgemeinen Formel I erfolgt nach bekannten Methoden.

Die Verseifung der Acylgruppen erfolgt beispielsweise mit Alkali oder Erdalkali-carbonaten oder -hydroxyden in einem Alkohol oder der wäßrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, vorzugsweise jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei − 10 °C bis 70 °C, vorzugsweise bei 25 °C.

Die Umsetzung der Verbindung der allgemeinen Formel I mit $R_3$ in der Bedeutung eines Wasserstoffatoms mit einem Isocyanat der allgemeinen Formel IV erfolgt gegebenenfalls unter Zusatz eines tertiären Amins, wie zum Beispiel Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Benzol, Toluol, Dimethylsulfoxid, bei Temperaturen ober- oder unterhalb Raumtemperatur, zum Beispiel zwischen − 80 °C bis 100 °C, vorzugsweise bei 0 °C bis 30 °C, vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmäßigerweise von Ausgangsprodukten aus, in denen diese durch vorzugsweise leicht abspaltbare Äther- oder Acylreste intermediär geschützt sind.

Die Oxydation der 15-OH-Gruppe mit Braunstein ist in «Reagents for organic Synthesis», Fieser and Fieser Vol. 1, 637, J. Wiley N.Y. 1967, beschrieben. Die sich anschließende Grignardierung mit Methylmagnesiumhalogeniden wird nach den dem Fachmann bekannten Methoden vorgenommen.

Die als Ausgangsmaterial dienenden Verbindungen der allgemeinen Formel II können beispielsweise hergestellt werden, indem man in an sich bekannter Weise ein bekanntes Prostaglandin F-Derivat der allgemeinen Formel V

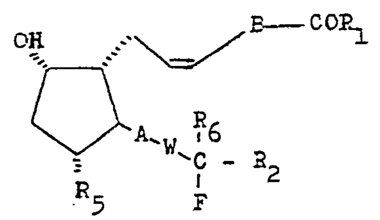

(V)

mit Jod in Gegenwart eines Alkalihydrogencarbonats oder Alkalicarbonats zu den Verbindungen der allgemeinen Formel VI

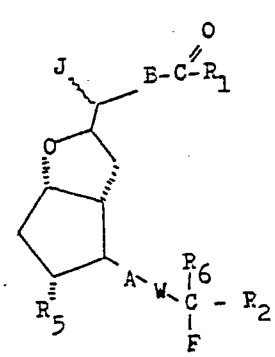

(VI)

worin in V und VI $R_1$, $R_2$, $R_5$, $R_6$, A, B und W die obige Bedeutung haben, umsetzt.

Anschließend kann man gegebenenfalls freie Hydroxygruppen durch Veresterung oder Silylierung schützen. Je nach der gewünschten Bedeutung von A oder $R_1$ in den Endprodukten der allgemeinen Formel I kann man gegebenenfalls Doppelbindungen in VI hydrieren oder gegebenenfalls eine Carboxygruppe verestern oder eine Carboxygruppe mit Verbindungen der allgemeinen Formel IV umsetzen.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0 °C und 80 °C durchgeführt.

Die Umsetzung der Verbindungen der allgemeinen Formel VI zu den Verbindungen der allgemeinen Formel II kann beispielsweise mit 1,5-Diazabicyclo [4.3.0] nonen-5 (DBN) oder 1,5-Diazabicyclo [5.4.0]-undecen-5 (DBU) in einem inerten Lösungsmittel wie Benzol, Toluol, Tetrahydrofuran usw. oder mit Natriummethylat in Methanol erfolgen. Die Halogenwasserstoffabspaltung wird bei Temperaturen zwischen 0 °C und 120 °C, vorzugsweise bei 20-60 °C durchgeführt.

Die neuen Prostacyclinderivate der allgemeinen Formel I sind wertvolle Pharmaka, da sie bei ähnlichem Wirkungsspektrum gegenüber entsprechenden Prostaglandinen durch bessere Spezifität und vor allem durch eine wesentlich längere Wirkung auszeichnen. Im Vergleich zu PGE, PGA und PGI zeichnen sich die neuen Prostaglandine durch größere Stabilität aus. Die gute Gewebsspezifität der neuen Prostaglandine zeigt sich bei der Untersuchung an glattmuskulären Organen wie z. B. am Meerschweinchenileum oder am Meerschweinchenuterus, wo eine wesentlich geringere Stimulation zu beobachten ist als bei Applikation natürlicher Prostaglandine.

Die neuen Prostaglandin-Analoga besitzen die für die Prostaglandine typischen pharmakologischen Eigenschaften, wie beispielsweise Senkung des Blutdrucks, Inhibierung der Thrombozytenaggregation, Hemmung der Magensäuresekretion, bronchodilatatorische Eigenschaften u.a. Die Verabreichung der beanspruchten Verbindungen für die obengenannten Wirkungsweisen erfolgt in Dosisbereichen von 5 bis 5 000 µg/kg/Tag.

Die neuen Prostacyclinderivate der allgemeinen Formel I besitzen gegenüber $PGI_2$ eine erhöhte Stabilität. Bei vergleichbarer Ulcus-Inhibierung bei der Indomethacin-induzierten Ulceration haben die neuen Verbindungen einen geringeren Einfluß auf Blutdruck und Thrombozytenaggregation als $PGI_2$.

Bei intravenöser Injektion an wachen, hypertonen Ratten in Dosen von 10 bis 150 µg/kg Körpergewicht zeigen die erfindungsgemäßen Verbindungen eine stärkere blutdrucksenkende Wirkung als $PGE_2$ und $PGA_2$, ohne wie $PGE_2$, in diesen Dosierungen Durchfälle oder wie $PGA_2$ kardiale Arrhythmien auszulösen.

Bei intravenöser Injektion an narkotisierten Kaninchen zeigen die erfindungsgemäßen Verbindungen im Vergleich zu $PGE_2$ und $PGA_2$ eine stärkere und erheblich länger anhaltende Blutdrucksenkung ohne daß andere glattmuskuläre Organe oder Organfunktionen beeinflußt werden.

Für die Asthma-Therapie sind Aerosol-Zubereitungen vorgesehen.

Für die parenterale Verabreichung werden sterile, injizierbare, wäßrige oder ölige Lösung benutzt.

Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen, zum Beispiel zur Herstellung von Blutdrucksenkern und Asthma-Mittel dienen.

## Beispiel 1

5-Cyano-16-fluor-prostacyclin-methylester-11,15-diacetat

Zu einer Lösung von 773 mg 16-Fluor-prostacyclin-methylester-11,15-diacetat in 5 ml Äther tropft man unter Argon bei − 70 °C 262 mg Chlorsulfonylisocyanat gelöst in 5 ml Äther. Nach 15 Minuten wird mit 6 ml Dimethylformamid versetzt und 2 Stunden bei 20° gerührt. Anschließend verdünnt man mit 100 ml eiskalter 5 %iger Natriumhydrogenkarbonatlösung und extrahiert dreimal mit je 100 ml Äther. Die organische Phase wird mit 30 ml Sole geschüttelt, über Magnesiumsulfat getrocknet und im Vakuum eingedampft. Zur Reinigung chromatographiert man an einer Kieselgelsäule, wobei mit Hexan-Äther-Gemischen eluiert wird. Dabei erhält man 430 mg der Titelverbindung als Öl.

IR : 2 955, 2 858, 2 200, 1 730, 1 650, 1 280, 970/cm.

Das Ausgangsmaterial für die Titelverbindung wird wie folgt hergestellt :

1a) 5,6-Dihydro-16-fluor-5-jod-prostacyclin-methylester

Eine Mischung aus 2 g 16-Fluor-prostaglandin-$F_{2\alpha}$-methylester (hergestellt nach DE-OS 23 20 368) 5,3 g Natriumhydrogenkarbonat, 50 ml Äther und 90 ml Wasser versetzt man bei 0 °C innerhalb von 3 Stunden unter Rühren mit 65 ml einer 2,5 %igen ätherischen Jodlösung. Man rührt 22 Stunden bei 0 °C, verdünnt mit 250 ml Äther, schüttelt mit Natriumthiosulfatlösung und Sole, trocknet über Magnesiumsulfat und dampft im Vakuum ein. Man erhält 2,5 g der Titelverbindung als Öl.
IR : 3 600. 3 420 (breit), 2 955, 1 730, 972/cm.

1b) 5,6-Dihydro-16-fluor-5-jod-prostacyclin-methylester-11,15-diacetat

Man löst 2,5 g der nach Beispiel la hergestellten Verbindung mit 10 ml Pyridin und 5 ml Essigsäureanhydrid, läßt 18 Stunden bei 20° stehen, dampft im Vakuum ein, versetzt mit 5 ml Toluol, dampft erneut ein und erhält 3,1 g der Titelverbindung als Öl.
IR : 2 955, 1 735, 1 260, 974/cm.

1c) 16-Fluor-prostacyclin-methylester-11,15-diacetat

Man löst 1,20 g der nach Beispiel 1 b hergestellten Verbindung in 12 ml Benzol, versetzt mit 6 ml 1,5-Diazabicyclo [4.3.0] non-5-en und erhitzt unter Argon 24 Stunden auf 50 °C. Man kühlt ab, verdünnt mit 400 ml Äther, schüttelt dreimal mit je 15 ml Eiswasser, trocknet über Natriumsulfat und dampft bei 25° im Vakuum ein. Das Dünnschichtchromatogramm an Kieselgelplatten (Laufmittel : Äther) zeigt einen Hauptfleck ($R_F$-Wert 0,62) und eine geringfügige polarere Verunreinigung ($R_F$-Wert 0,45). Ausbeute : 1,02 g. Das Präparat wird ohne weitere Reinigung verwendet.

Beispiel 2

5-Cyano-16-fluor-prostacyclin-methylester

400 mg 5-Cyano-16-fluor-prostacyclin-methylester-11,15-diacetat (hergestellt nach Beispiel 1), 240 mg Kaliumkarbonat und 15 ml Methanol werden 3 Stunden bei 25° unter Argon gerührt. Anschliessend verdünntt man mit 200 ml Äther, wäscht dreimal mit je 20 ml Wasser, trocknet über Natriumsulfat und dampft im Vakuum ein. Man erhält 290 mg der Titelverbindung als Öl.
IR : 3 600, 3 420, 2 950, 2 862, 2 205, 1 735, 1 650, 974/cm.

Beispiel 3

5-Cyano-16-fluor-prostacyclin

Zu einer Lösung von 120 mg 5-Cyano-16-fluor-prostacyclin-methylester (siehe Beispiel 2) in 10 ml Methanol gibt man 1 ml 1 N Natronlauge, rührt 2 Stunden bei 25 °C, dampft im Vakuum ein, versetzt mit 20 ml Sole und 10 ml Zitratpuffer (pH 6,5) und extrahiert viermal mit je 25 ml Methylenchlorid. Die organische Phase wird mit 10 ml Sole geschüttelt, über Magnesiumsulfat getrocknet und im Vakuum bei 25° eingedampft. Man erhält 100 mg der Titelverbindung als Öl. Das Dünnschichtchromatogramm an Kieselgelplatten mit dem Laufmittel Methylenchlorid/Methanol (8 + 2) zeigt nur einen Fleck mit dem $R_F$-Wert 0,42.
IR : 3 602, 3 420, 2 948, 2 858, 2 205, 1 710, 1 650, 976/cm.

Beispiel 4

5-Cyano-16-fluor-20-methyl-prostacyclin-methylester-11,15-diacetat

Man löst 485 mg 16-Fluor-20-methyl-prostacyclin-methylester-11,15-diacetat in 4 ml Äther und tropft unter Argon bei − 70° eine Lösung von 169 mg Chlorsulfonylisocyanat in 3 ml Äther zu, rührt 10 Minuten, versetzt mit 3 ml Dimethylformamid und rührt 2 Stunden bei 20°. Anschließend verdünnt man mit 60 ml eiskalter Natriumhydrogenkarbonatlösung (5 %ig), extrahiert dreimal mit je 75 ml Äther, wäscht die organische Phase mit 20 ml Sole, trocknet über Magnesiumsulfat und dampft im Vakuum ein. Man reinigt durch Chromatographie an Kieselgel, mit Hexan/Äther (2 : 1) werden 300 mg der öligen Titelverbindung eluiert.
IR : 2 950, 2 860, 2 205, 1 730, 1 650, 1 265, 972/cm.
Das Ausgangsmaterial für die Titelverbindung wird wie folgt hergestellt :

4a) 5,6-Dihydro-16-fluor-5-jod-20-methyl-prostacyclin-methylester

Man löst 1,5 g 16-Fluor-20-methyl-prostaglandin-methylester (hergestellt nach DE-OS 23 20 368) in 35 ml Äther, versetzt mit 70 ml Wasser und 4 g Natriumhydrogencarbonat und tropft unter Rühren bei 0° innerhalb von 2 Stunden. 48 ml einer 2,5 %igen ätherischen Jodlösung zu. Man rührt weitere 20 Stunden bei 0°, verdünnt mit 200 ml Äther, schüttelt nacheinander mit Natriumthiosulfatlösung und Sole, trocknet über Magnesiumsulfat und dampft im Vakuum ein. Man erhält 1,95 g der Titelverbindung als Öl.
IR : 3 605, 3 400 (breit), 2 956, 1 735, 974/cm.

4b) 5,6-Dihydro-16-fluor-5-jod-20-methyl-prostacyclin-methylester-11,15-diacetat

Man verfährt wie in Beispiel 1b und erhält aus 1,90 g des nach Beispiel 4a hergestellten Diols 2,35 rohes Diacetat, das durch Chromatographie an Kieselgel gereinigt wird. Durch Elution mit Hexan/Essigester (1 : 1) erhält man 2,05 g der reinen Titelverbindung als Öl.
IR : 2 952, 1 735, 1 275, 978/cm.

4c) 16-Fluor-20-methyl-prostacyclin-methylester-11,15-diacetat

Eine Lösung von 1,5 mg des nach Beispiel 4b hergestellten Diacetats in 15 ml Benzol und 8 ml 1,5 Diazabicyclo [4.3.0]-non-5-en erhitzt man 18 Stunden auf 55° unter Argon. Anschließend kühlt man auf 0° ab, verdünnt mit 500 ml Äther, schüttelt mehrmals mit wenig Eiswasser aus, trocknet über Natriumsulfat und dampft bei 25° im Vakuum ein. Man erhält 1,2 g der Titelverbindung als Öl, das ohne weitere Reinigung zur Einführung der Nitrilgruppe verwendet wird.

Beispiel 5

5-Cyano-16-fluor-20-methyl-prostacyclin-methylester

200 mg 5-Cyano-16-fluor-20-methyl-prostacyclin-methylester-11,15-diacetat gelöst in 8 ml Methanol werden mit 220 mg Kaliumcarbonat 3 Stunden bei 20-25° gerührt. Anschließend wird mit 150 ml Äther verdünnt, dreimal mit je 15 ml Wasser geschüttelt, über Natriumsulfat getrocknet und im Vakuum eingedampft. Man erhält 140 mg der Titelverbindung als Öl.
IR : 3 600, 3 410, 2 962, 2 860, 2 205, 1 735, 1 652, 976/cm.

Beispiel 6

5-Cyano-16-fluor-20-methyl-prostacyclin

Man löst 100 mg 5-Cyano-16-fluor-20-methyl-prostacyclin-methylester (hergestellt nach Beispiel 5) in 5 ml Methanol, versetzt mit 1 ml 1 N Natronlauge und rührt 2 Stunden bei 25° unter Argon. Anschließend dampft man im Vakuum ein, löst den Rückstand in 20 ml Sole, versetzt mit 10 ml Zitratpuffer (pH 6,5), extrahiert mehrmals mit Methylenchlorid, wäscht die organische Phase einmal mit wenig Sole, trocknet über Magnesiumsulfat und dampft im Vakuum ein. Man erhält 85 mg der Titelverbindung als Öl.
IR : 3 600, 3 420, 2 954, 2 860, 2 202, 1 708, 1 650, 978/cm.
Das Dünnschichtchromatogramm der Substanz an Kieselgelplatten zeigt mit Methylenchlorid-Methanol (8 + 2) als Laufmittel nur einen Fleck, $R_F$-Wert : 0,47.

Beispiel 7

5-Cyano-16-fluor-15-methyl-prostacyclin

Eine Lösung von 200 mg 5-Cyano-15-dehydro-16-fluor-prostacyclinmethylester in einem Gemisch aus 5 ml Äther und 5 ml Tetrahydrofuran wird bei − 100° unter Argon mit 1,5 ml einer 1 m ätherischen Methylmagnesiumbromidlösung innerhalb von 20 Minuten versetzt. Nach weiteren 20 Minuten bei − 70° werden Eisstückchen zugegeben, auf 0° erwärmt, mit Zitratpuffer (pH 6) versetzt und mehrmals mit Methylenchlorid extrahiert. Die vereinigten Extrakte werden mit wenig Sole gewaschen und im Vakuum eingedampft. Den Rückstand löst man in 5 ml Methanol, versetzt mit 1 ml 1 N Natronlauge, rührt zwei Stunden bei 20° und dampft im Vakuum ein. Anschließend wird mit Zitratpuffer (pH 6) verdünnt, mit Methylenchlorid extrahiert, der Extrakt mit Sole gewaschen, über Magnesiumsulfat getrocknet und im Vakuum eingedampft. Der Rückstand wird durch Chromatographie an Kieselgel gereinigt. Mit Methylenchlorid/5 % Isopropanol eluiert man 80 mg der Titelverbindung als Öl.
IR : 3 600, 3 420, 2 952, 2 860, 2 202, 1 708, 1 650, 978/cm.
Das Ausgangsmaterial für die Titelverbindung wird wie folgt hergestellt :

7a) 5-Cyano-15-dehydro-16-fluor-prostacyclin-methylester

Eine Lösung von 500 mg 5-Cyano-16-fluor-prostacyclin-methylester in 20 ml Äther wird mit 5 g Braunstein versetzt und vier Stunden bei 20° gerührt. Diese Aufschlämmung gibt man auf eine Kieselgelsäule (1,5 × 20 cm) und wäscht mit 200 ml Äther nach. Nach Eindampfen des Eluats erhält man 220 mg der Titelverbindung als Öl.
IR : 3 600, 2 950, 2 862, 2 205, 1 732, 1 695, 1 628, 978/cm.

Beispiel 8

5-Cyano-16-fluor-16-methyl-prostacyclin

Eine Lösung von 255 mg 3-Fluor-3-methyl-2-oxo-heptanphosphonsäuredimethylester in 10 ml Dimethoxyäthan tropft man zu einer Suspension von 48 mg 50 %igem Natriumhydrid in 5 ml Dimethoxyäthan unter Argon, rührt eine Stude bei 20°, kühlt dann auf 0° und tropft eine Lösung von 320 mg 5-{(E)-(1S, 5R, 6R, 7R)-7-Acetoxy-6-formyl-2-oxabicyclo [3.3.0] octan-3-yliden}-5-cyano-pentansäuremethylester in 5 ml Dimethoxyäthan innerhalb von 5 Minuten zu, rührt eine weitere Stunde bei 10°, verdünnt mit 150 ml Citratpuffer (pH 6), extrahiert dreimal mit je 40 ml Äther, wäscht die organische Phase mit 10 ml Sole, trocknet über Magnesiumsulfat und dampft im Vakuum ein. Den Rückstand löst man in 20 ml einer Äthanol-Tetrahydrofuranmischung (1 : 1), kühlt auf − 40° und versetzt mit 1 g Natriumborhydrid. Unter Rühren beläßt man die Temperatur bei − 40° und verfolgt den Verlauf der Reaktion durch Dünnschichtchromatographie an Kieselgelplatten (Laufmittel : Toluol-Essigester 7 + 3). Nach zwei Stunden wird mit 100 ml Citratpuffer (pH 6) verdünnt, dreimal mit je 40 ml Methylenchlorid extrahiert, die vereinigten Extrakte mit 10 ml Sole geschüttelt, über Magnesiumsulfat getrocknet und im Vakuum eingedampft. Das Dünnschichtchromatogramm (Toluol-Essigester 8 + 2, zweimal gelaufen) zeigt zwei Flecken der an C-15 epimeren Alkohole mit den $R_F$-Werten 0,39 und 0,45. Zur Trennung chromatographiert man an einer Kieselgelsäule (1,5 × 50 cm) und eluiert mit Hexan und steigendem Essigestergradienten. Man erhält 140 mg 5-Cyano-16-fluor-16-methyl-prostacyclin-methylester als Öl. Zur Verseifung löst man in 4 ml Methanol und 1 ml 1 N Natronlauge, rührt zwei Stunden bei 25°, dampft bei 25° im Vakuum den größten Teil des Lösungsmittels ab, verdünnt mit 50 ml Citratpuffer (pH 6), extrahiert dreimal mit je 30 ml Methylenchlorid, wäscht die vereinigte organische Phase mit 10 ml Sole, trocknet über Magnesiumsulfat und dampft im Vakuum ein. Man erhält 115 mg der Titelverbindung als Öl.

IR : 3 600, 3 420, 2 952, 2 860, 2 204, 1 708, 1 650, 978/cm.

Das Ausgangsmaterial für die Titelverbindung wird wie folgt hergestellt :

8a) (1S, 5R, 6S, 7R)-7-Benzoyloxy-6-(tert.-butyl-dimethylsilyloxy)-methyl-2-oxabicyclo [3.3.0] octan-3-on

Eine Lösung von 16,58 g (1S, 5R, 6S, 7R)-7-Benzoyloxy-6-hydroxymethyl-2-oxabicyclo [3.3.0] octan-3-on in 18 ml Dimethylformamid wird bei 0° mit 10,21 g Imidazol und 10,85 g tert.-Butyldimethylsilylchlorid versetzt. Man rührt 24 Std. bei 20°, verdünnt mit 1,5 l Äther, schüttelt nacheinander mit 5 %iger Schwefelsäure und Sole, trocknet über Magnesiumsulfat und dampft im Vakuum ein. Der Rückstand wird aus Hexan umkristallisiert. Man erhält 20 g farblose Kristalle, Fp. 75°.

IR : 2 955, 2 930, 2 858, 1 770, 1 712, 1 272, 1 108, 834/cm.

8b) (1S, 3RS, 5R, 6S, 7R)-6-(tert.-butyl-dimethylsilyloxy)-methyl-2-oxabicyclo [3.3.0] octan-3.7-diol

Zu einer Lösung von 10 g der nach Beispiel 8a hergestellten Verbindung in 400 ml Toluol tropft man unter Rühren bei − 70° 75 ml einer 20 %igen Lösung von Diisobutylaluminiumhydrid in Toluol innerhalb von 20 Minuten, nach weiteren 15 Minuten versetzt man mit 5 ml Isopropanol, dann mit 40 ml Wasser und rührt eine Stunde bei Raumtemperatur. Anschließend wird filtriert, der Niederschlag mit Methylenchlorid nachgewaschen und die Lösung im Vakuum eingedampft. Zur Entfernung von Benzaldehyd und Benzylalkohol wird mit Hexan-Essigester-Gemischen über Kieselgel chromatographiert. Man erhält 7,1 g der Titelverbindung als Öl.

IR : 3 600, 3 410, 2 950, 2 860, 1 110, 835/cm.

8c) (5Z)-7-[(1R, 2S, 3R, 5S)-2-(tert.-butyldimethylsilyloxy)-methyl-3,5-dihydroxy-cyclopentyl]-5-heptensäuremethylester

11,52 g 50 %iges Natriumhydrid werden durch Behandeln mit Pentan vom Mineralöl befreit und mit 100 ml Dimethylsulfoxid bei 70° 1,5 Stunden gerührt. Die so erhaltene Lösung von Dimsylnatrium wird zu 53,4 g 4-Carboxybutyl-triphenylphosphoniumbromid gelöst in 400 ml Dimethylsulfoxid getropft und anschließend 30 Minuten bei 20° gerührt. Zu der so erhaltenen orangeroten Ylenlösung tropft man eine Lösung von 7 g der nach Beispiel 8b erhaltenen Verbindung in 100 ml Dimethylsulfoxid und rührt drei Stunden bei 50°. Zur Aufarbeitung verdünnt man mit 3 l Sole und extrahiert mit 500 ml Äther. Dieser Extrakt enthält hauptsächlich Triphenylphosphinoxid. Die wässrige Phase wird mit Zitronensäure auf pH 5 eingestellt und dreimal mit Äther extrahiert, der Extrakt mit Sole geschüttelt und über Magnesiumsulfat getrocknet, im Vakuum eingedampft. Der Rückstand wird zur Veresterung in Methylenchlorid gelöst und bei 0° mit ätherischer Diazomethanlösung behandelt, anschließend im Vakuum eingedampft. Zur Reinigung chromatographiert man an Kieselgel mit Methylenchlorid und erhält 7,9 g der Titelverbindung als Öl.

IR : 3 600, 1 735, 1 110, 835/cm.

8d) (1S, 3RS, 5R, 6S, 7R)-3-[(1RS)-1-Jod-4-methoxycarbonyl-1-butyl]-6-(tert.-butyldimethylsilyloxy)-methyl-2-oxabicyclo [3.3.0] octan-7-ol

Zu einer Mischung aus 7 g der nach Beispiel 8c erhaltenen Verbindung, 25 g Natriumhydrogencarbonat, 200 ml Äther und 200 ml Wasser tropft man unter rühren bei 0° innerhalb von 4 Stunden eine Lösung von 6,1 g Jod in 300 ml Äther, rührt dannweitere 20 Stunden bei 0°, schüttelt nacheinander mit Natriumthiosulfatlösung und Sole, trocknet über Magnesiumsulfat und dampft im Vakuum ein. Man erhält 9 g der öligen Titelverbindung.

IR : 3 600, 2 955, 2 860, 1 732, 1 105, 834/cm.

8e) (1S, 3RS, 5R, 6S, 7R)-3-[(1RS)-1-Jod-4-methoxycarbonyl-1-butyl]-6-(tert.-butyldimethylsilyloxy)-methyl-7-acetoxy-2-oxabicyclo [3.3.0] octan

Man löst 8 g der nach Beispiel 8d erhaltenen Verbindung in 20 ml Pyridin, versetzt mit 8 ml Essigsäureanhydrid und läßt 20 Stunden bei 20° stehen. Anschließend dampft man im Vakuum ein und reinigt den Rückstand durch Chromatographie an Kieselgel mit Hexan-Essigester (3 : 1). Man erhält 7,5 g der Titelverbindung als Öl.

IR : 2 960, 2 858, 1 735, 1 105, 834/cm.

8f) 5-{(Z)-(1S, 5R, 6S, 7R)-7-Acetoxy-6-(tert.-butyldimethylsilyloxy)-methyl-2-oxabicyclo [3.3.0] octan-3-yliden}-pentansäure-methylester

Man löst 7,5 g der nach Beispiel 8e hergestellten Verbindung in 100 ml Toluol und 50 ml 1,5-Diazabicyclo [4.3.0] non-5-en und erhitzt unter Argon 20 Stunden auf 50°. Man kühlt ab, verdünnt mit 1 l Äther und schüttelt dreimal mit je 75 ml Eiswasser aus, trocknet über Magnesiumsulfat und dampft bei 25° im Vakuum ein.

8g) 5-{(E)-(1S, 5R, 6S, 7R)-7-Acetoxy-6-(tert.-butyldimethylsilyloxy)-methyl-2-oxabicyclo [3.3.0] octan-3-yliden}-5-cyanopentansäuremethylester

Zu einer Lösung der nach Beispiel 8f erhaltenen Substanz in 75 ml Äther tropft man bei − 70° unter Argon 2,75 g Chlorsulfonylisocyanat gelöst in 50 ml Äther innerhalb von 45 Minuten, versetzt dann mit 50 ml Dimethylformamid und nach 10 Minuten mit 10 ml Triäthylamin und rührt anschließend 1 Stunde bei − 10°, verdünnt dann mit 500 ml 5 %iger Natriumhydrogencarbonatlösung und extrahiert dreimal mit je 200 ml Äther. Die organische Phase wird mit 50 ml Sole geschüttelt, über Magnesiumsulfat getrocknet und im Vakuum bei 25° eingedampft. Man reinigt durch Chromatographie an Kieselgel, wobei mit Hexan-Äther-Gemischen 1,95 g der Titelverbindung als Öl eluiert werden.

IR : 2 955, 2 862, 2 210, 1 735, 1 648, 1 265, 1 110, 834/cm.

8h) 5-{(E)-(1S, 5R, 6S, 7R)-7-Acetoxy-6-hydroxymethyl-2-oxabicyclo [3.3.0] octan-3-yliden}-5-cyano-pentansäuremethylester

Zu einer Lösung von 1,50 g der nach Beispiel 8 g hergestellten Verbindung in 50 ml Tetrahydrofuran gibt man 800 mg Tetrabutylammoniumfluorid und rührt 4 Stunden bei 20°. Anschliessend wird mit 100 ml Äther verdünnt, dreimal mit je 10 ml Sole geschüttelt, über Magnesiumsulfat getrocknet und im Vakuum eingedampft. Den Rückstand reinigt man durch Chromatographie an Kieselgel. Mit Hexan-Essigester (1 : 2) eluiert man 800 mg der Titelverbindung als Öl.

IR : 3 580, 2 950, 2 862, 2 208, 1 735, 1 648, 1 262/cm.

8i) 5-{(E)-(1S, 5R, 6R, 7R)-7-Acetoxy-6-formyl-2-oxabicyclo [3.3.0]-octan-3-yliden}-5-cyano-pentansäuremethylester

Man löst 3,5 g Collins-Reagenz (Organic Synthesis *52* 5) in 50 ml Methylenchlorid. Unter Rühren versetzt man bei 10° mit einer Lösung von 800 mg der nach Beispiel 8h hergestellten Verbindung in 10 ml Methylenchlorid. Man rührt noch 15 Minuten nach, verdünnt mit 200 ml Äther, dekantiert und schüttelt die organische Phase nacheinander zweimal mit je 20 ml 10 %iger Schwefelsäure, zweimal mit je 20 ml 5 %iger Natriumhydrogencarbonatlösung und zweimal mit 20 ml Sole, trocknet über Magnesiumsulfat und dampft bei 25° im Vakuum ein. Man erhält 620 mg der Titelverbindung als Öl.

IR : 2 952, 2 860, 2 740, 2 205, 1 730, 1 720 (Schulter), 1 648, 1 260/cm.

Beispiel 9

5-Cyano-16-fluor-N-methansulfonyl-prostacyclin-carboxamid

Man löst 400 mg 5-Cyano-16-fluor-prostacyclin (siehe Beispiel 3) in 2 ml Pyridin und 1 ml Essigsäureanhydrid und läßt 12 Stunden bei 25° stehen und dampft im Vakuum ein. Den Rückstand löst man in 10 ml Acetonitril, versetzt mit 130 mg Triäthylamin und anschliessend mit einer Lösung von 160 mg Methylsulfonylisocyanat in 10 ml Acetonitril. Nach 4 Stunden engt man im Vakuum ein, versetzt mit Zitratpuffer (pH 6) und extrahiert mit Äther. Man schüttelt die organische Phase mit Sole, trocknet über

Magnesiumsulfat und dampft im Vakuum ein. Der Rückstand wird in 10 ml Methanol gelöst und 2 Stunden mit 250 mg Kaliumcarbonat gerührt. Anschließend verdünnt man mit Sole, stellt mit Zitronsäure auf pH 6 ein und extrahiert mehrmals mit Methylenchlorid, trocknet über Magnesiumsulfat und dampft im Vakuum ein. Der Rückstand wird durch Chromatographie an Kieselgel gereinigt. Man eluiert mit Methylenchlorid/5 % Isopropanol und erhält 150 mg der Titelverbindung als Öl.

IR : 3 410, 2 950, 2 862, 2 205, 1 718, 1 650, 978/cm.

Beispiel 10

5-Cyano-16-fluor-20-methyl-N-acetyl-prostacyclin-carboxamid

205 mg 5-Cyano-16-fluor-20-methyl-prostacyclin (Herstellung siehe Beispiel 6), 1,5 ml Pyridin und 0,5 ml Essigsäureanhydrid rührt man 16 Stunden bei 20° und dampft im Vakuum ein. Den Rückstand löst man in 6 ml Acetonitril, versetzt mit 80 mg Triäthylamin und tropft bei 0° eine Lösung von 58 mg Acetylisocyanat in 10 ml Acetonitril zu. Anschließend rührt man 2 Stunden bei 20°, engt im Vakuum ein, verdünnt mit 50 ml Citratpuffer (pH 6,5) und extrahiert dreimal mit je 40 ml Äther. Die vereinigten Extrakte werden mit 10 ml Sole geschüttelt, über Magnesiumsulfat getrocknet und im Vakuum eingedampft. Zur Abspaltung der Acetatgruppen löst man in 5 ml Methanol und rührt 2 Stunden bei 20° mit 110 mg Kaliumcarbonat, verdünnt dann mit 75 ml Sole, stellt durch Zugabe von Zitronensäure pH 6,5 ein und extrahiert mehrmals mit je 30 ml Methylenchlorid, wäscht den Extrakt mit 10 ml Sole und trocknet über Magnesiumsulfat. Den Eindampfrückstand chromatographiert man an Kieselgel, wobei mit Methylenchlorid/1 % Isopropylalkohol eluiert wird. Man erhält 85 mg der Titelverbindung als Öl.

IR : 3 600, 3 410, 2 960, 2 858, 2 204, 1 735, 1 705, 1 648, 976/cm.

Beispiel 11

5-Cyano-16,16-difluor-prostacyclin

Eine Lösung von 260 mg 3,3-Difluor-2-oxo-heptanphosphonsäuredimethylester [Prostaglandins 9 527 (1975)] in 10 ml Dimethoxyäthan tropft man zu einer Suspension von 48 mg Natriumhydrid (50 %ige Mischung mit Mineralöl) in 5 ml Dimethoxyäthan unter Argon. Man rührt eine Stunde bei 0°, tropft dann bei 0° eine Lösung von 320 mg 5-{(E)-(1S, 5R, 6R, 7R)-7-Acetoxy-6-formyl-2-oxabicyclo [3.3.0] octan-3-yliden}-5-cyano-pentansäuremethylester (siehe Beispiel 8i) in 5 ml, Dimethoxyäthan innerhalb von 5 Minuten zu, rührt eine weitere Stunde bei 20°, verdünnt mit Citratpuffer (pH 6), extrahiert mit Äther, wäscht die organische Phase mit Sole, trocknet über Magnesiumsulfat und dampft im Vakuum ein und erhält 15-Dehydro-16,16-difluor-5-cyano-prostacyclin-methylester-11-acetat. Zur Reduktion der 15-Ketogruppe löst man den Rückstand in 10 ml Methanol und versetzt bei – 40° mit 0,8 g Natriumborhydrid. Man rührt zwei Stunden bei – 40°, verdünnt mit Citratpuffer (pH 5) und extrahiert mit Methylenchlorid, wäscht die organische Phase mit Sole, trocknet über Magnesiumsulfat und dampft im Vakuum ein. Den Rückstand behandelt man in Methanol mit 1 N Natronlauge (siehe Beispiel 3) und reinigt das so erhaltene Produkt durch präparative Dünnschichtchromatographie an Kieselgelplatten (2,5 mm), die mit Chloroform/Isopropanol (7 + 3) entwickelt werden. Die polarere Zone wird von der Platte gekratzt und mit Chloroform/Isopropanol (9 + 1) eluiert. Dabei erhält man 90 mg der Titelverbindung als Öl.

IR :3 600, 3 410, 2 955, 2 862, 2 210, 1 710, 1 652, 972/cm.

**Ansprüche**

1. Prostacyclinderivate der allgemeinen Formel I

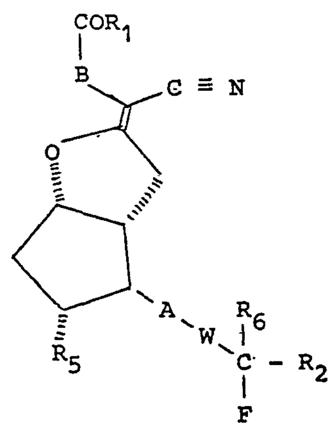

(I)

worin

$R_1$ den $OR_3$, wobei $R_3$, Wasserstoff oder Alkyl mit 1-10 C-Atomen bedeuten kann, oder $R_1$ den Rest $NHR_4$ mit $R_4$ in der Bedeutung eines Alkanoylrestes mit 1-4 C-Atomen oder eines Alkansulfonylrestes mit 1-4 C-Atomen darstellt,

B eine geradkettige oder verzweigtkettige Alkylgruppe mit 1-5 C-Atomen,

A eine $-CH_2-CH_2-$, cis-CH=CH- oder trans-CH=CH-Gruppe,

W eine Hydroxymethylengruppe oder eine

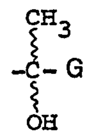 Gruppe,

in denen die OH-Gruppe jeweils durch Alkanoyl mit 1-4 C-Atomen substituiert sein kann, wobei die freie oder subtituierte OH-Gruppe α- oder β-ständig sein kann,

$R_2$ eine gesättigte gerad-oder verzweigtkettige Alkylgruppe mit 1-6 C-Atomen, die durch Phenyl substituiert sein kann oder eine Phenylgruppe,

$R_5$ eine Hydroxygruppe, die durch Alkanoyl mit 1-4 C-Atomen substituiert sein kann,

$R_6$ Wasserstoff, Fluor oder Alkyl mit 1-4 C-Atomen und falls $R_3$ Wasserstoff darstellt, deren Salze mit physiologisch verträglichen Basen bedeuten.

2. Verfahren zur Herstellung der Prostacyclinderivate der allgemeinen Formel I, gemäß Anspruch 1 dadurch gekennzeichnet, daß man eine Verbindung der allgemeinen Formel II,

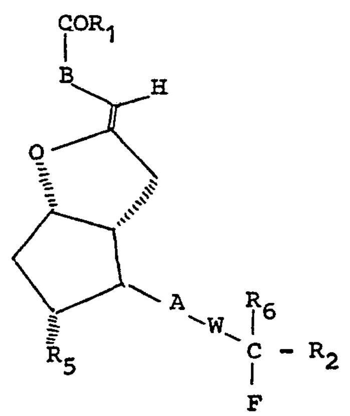

(II)

worin $R_1$, $R_2$, $R_5$, $R_6$, A, B und W die obenangegebenen Bedeutungen haben, gegebenenfalls nach Schutz anwesender freier Hydroxygruppen, mit einem Sulfonylisocyanat der allgemeinen Formell III,

$$R_7SO_2NCO \quad (III),$$

worin $R_7$ einen gegebenenfalls durch Alkyl mit 1-4 C-Atomen substituierten Phenylring, halogeniertes Alkyl oder Halogen bedeutet, und anschließend mit einem tertiären Amin oder tertiären Amid umsetzt und gegebenenfalls in den erhaltenen Verfahrensprodukten in beliebiger Reihenfolge geschützte Hydroxygruppen freisetzt und/oder freie Hydroxygruppen verestert, veräthert oder oxydiert und die erhaltenen Carbonylgruppen mit Methylmagnesiumhalogeniden umsetzt, eine veresterte Carboxylgruppe verseift oder eine Carboxylgruppe verestert, eine Carboxylgruppe mit Verbindungen der allgemeinen Formel IV

$$O{=}C{=}N{-}R_4 \quad (IV)$$

worin $R_4$ die oben angegebene Bedeutung hat, umsetzt oder eine Carboxylgruppe mit einer physiologisch verträglichen Base in ein Salz überführt.

3. Arzneimittel, enthaltend eine Verbindung des Anspruches 1 sowie übliche Hilfs- und Trägerstoffe.

4. 5-Cyano-16-fluor-prostacyclin

**Claims**

1. Prostacyclin derivatives of the general formula I

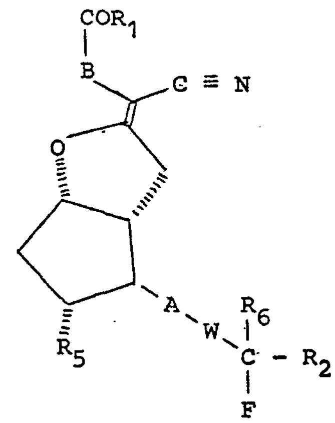

(I)

in which

$R_1$ represents the radical $OR_3$ in which $R_3$ represents hydrogen or alkyl having from 1 to 10 carbon atoms ; or $R_1$ represents the radical $NHR_4$ in which $R_4$ represents an alkanoyl radical having from 1 to 4 carbon atoms or an alkanesulphonyl radical having from 1 to 4 carbon atoms,

B represents a straight-chain or branched-chain alkyl group having from 1 to 5 carbon atoms,

A represents a $-CH_2-CH_2$-group, a cis-CH=CH-group or a trans-CH=CH-group,

W represents a hydroxymethylene group or a group

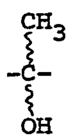

in which the OH group in each case can be substituted by alkanoyl having from 1 to 4 carbon atoms and in which the free or substituted OH groups can be in the α- or β-configuration,

$R_2$ represents a saturated straight-chain or branch-chain alkyl group having from 1 to 6 carbon atoms, which alkyl group can be substituted by phenyl or a phenyl group,

$R_5$ represents a hydroxy group which can be substituted by alkanoyl having from 1 to 4 carbon atoms, and

$R_6$ represents hydrogen, fluorine or alkyl having from 1 to 4 carbon atoms, and, if $R_3$ represents hydrogen, the salts thereof with physiologically tolerable bases.

2. Process for the manufacture of prostacyclin derivatives of the general formula I according to claim 1, characterised in that a compound of the general formula II

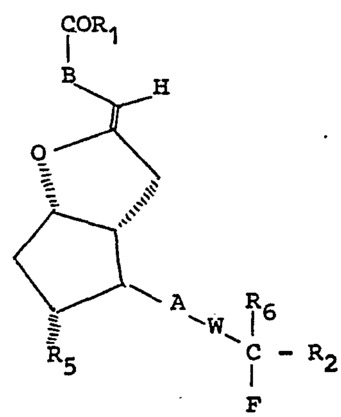

(II)

in which $R_1$, $R_2$, $R_5$, $R_6$, A, B and W have the meanings given above is reacted, optionally after protecting the free hydroxy groups present, with a sulphonyl isocyanate of the general formula III

$$R_7SO_2NCO \quad (III)$$

in which $R_7$ represents a phenyl ring optionally substituted by alkyl having from 1 to 4 carbon atoms, halogenated alkyl or halogen, and subsequently reacted with a tertiary amine or tertiary amide and, optionally, in the products resulting from the process, in any desired order, protected hydroxy groups are freed and/or free hydroxy groups are esterified or etherified or oxidised and the resulting carbonyl groups are reacted with methyl magnesium halides, an esterified carboxy group is hydrolysed or a carboxy group is esterified, a carboxy group is reacted with compounds of the general formula IV

$$O{=}C{-}N{=}R_4 \qquad (IV)$$

in which $R_4$ has the meaning given above, or a carboxy group is converted into a salt with a physiologically tolerable base.

3. Medicaments containing a compound of claim 1 and customary auxiliaries and carriers.

4. 5-cyano-16-fluoroprostacyclin.

**Revendications**

1. Dérivés de la prostacycline qui répondent à la formule générale I :

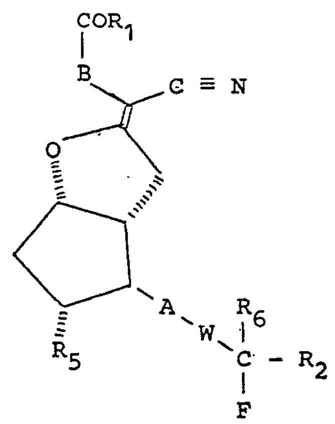

(I)

dans laquelle

$R_1$ représente un radical —$OR_3$ dans lequel $R_3$ désigne l'hydrogène ou un alkyle contenant de 1 à 10 atomes de carbone, ou encore $R_1$ représente un radical —$NHR_4$ dans lequel $R_4$ désigne un radical alcanoyle en $C_1$-$C_4$ ou un radical alcane-sulfonyle en $C_1$-$C_4$,

B représente un radical alkylène, linéaire ou ramifié, contenant de 1 à 5 atomes de carbone,

A représente un radical —$CH_2$—$CH_2$-, un radical —CH=CH- cis ou un radical —CH=CH- trans,

W représente un radical hydroxyméthylène ou un radical

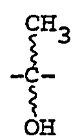

dans chacun desquels le groupe —OH peut porter comme substituant un alcanoyle en $C_1$-$C_4$, le groupe —OH libre ou substitué ayant la configuration $\alpha$ ou la configuration $\beta$,

$R_2$ représente un radical alkyle saturé, linéaire ou ramifié, contenant de 1 à 6 atomes de carbone et éventuellement porteur d'un phényle, ou représente un radical phényle,

$R_5$ représente un groupe hydroxy qui peut porter un alcanoyle en $C_1$-$C_4$, et

$R_6$ représente l'hydrogène, le fluor ou un alkyle en $C_1$-$C_4$, et également, dans le cas où $R_3$ désigne l'hydrogène, les sels que ces composés forment avec des bases acceptables du point de vue physiologique.

2. Procédé de préparation des dérivés de la prostacycline de formule générale I selon la revendication 1, procédé caractérisé en ce qu'on fait réagir un composé répondant à la formule générale II :

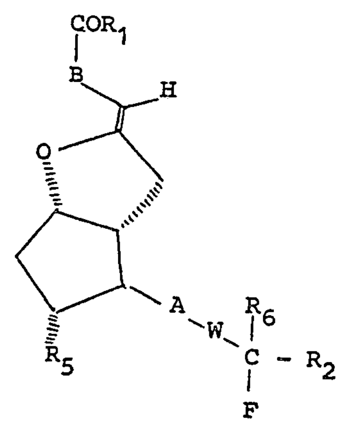

(II)

dans laquelle $R_1$, $R_2$, $R_5$, $R_6$, A, B et W ont les significations indiquées ci-dessus, éventuellement après avoir protégé d'éventuels groupes hydroxy libre, avec un isocyanate de sulfonyle répondant à la formule générale III ;

$$R_7SO_2NCO \qquad (III)$$

dans laquelle $R_7$ représente un radical phényle éventuellement porteur d'un alkyle en $C_1$-$C_4$, un alkyle halogéné ou un halogène, puis on fait réagir avec une amine tertiaire ou un amide tertiaire, et, le cas échéant, on effectue sur les produits obtenus, dans l'ordre que l'on veut, une ou plusieurs des réactions suivantes : on libère des groupes hydroxy protégés, on estérifie, éthérifie ou oxyde des groupes hydroxy libres, on fait réagir les radicaux carbonyles ainsi obtenus avec des halogénures de méthyl-magnésiums, on saponifie un radical carboxy estérifié, on estérifie un radical carboxy, on fait réagir un radical carboxy avec des composés répondant à la formule générale IV :

$$O{=}C{=}N{-}R_4 \qquad (IV)$$

dans laquelle $R_4$ a la signification précédemment donnée, et on transforme un radical carboxy en un sel avec une base acceptable du point de vue physiologique.

3. Médicament contenant un composé tel que spécifié à la revendication 1 ainsi que des adjuvants et excipients usuels.

4. Cyano-5 fluoro-16 prostacycline.